# EUROPÄISCHE PATENTSCHRIFT

(11) **EP 3 211 387 B1**
(45) Veröffentlichungstag und Bekanntmachung des Hinweises auf die Patenterteilung: **07.10.2020**
(21) Anmeldenummer: 17156890.0
(22) Anmeldetag: 20.02.2017
(51) Int. Cl.: G01F 1/32, G01F 1/66, G01N 33/28, G01F 1/704

(54) **MESSSTAB FÜR DEN NACHWEIS EINES STRÖMENDEN MEDIUMS IN EINEM ROHR UND DIESBEZÜGLICHE MESSANORDNUNG**
MEASURING ROD FOR THE DETECTION OF A FLOWING MEDIUM IN A TUBE AND RELATED MEASURING ASSEMBLY
JAUGE DE DÉTECTION D'UN MILIEU S'ÉCOULANT DANS UN TUBE ET SYSTÈME DE MESURE ASSOCIÉ

(30) Priorität: 26.02.2016 DE 102016103419
(43) Veröffentlichungstag der Anmeldung: 30.08.2017
(73) Patentinhaber: Krohne Messtechnik GmbH, 47058 Duisburg (DE)
(72) Erfinder: Drenthen, Jan, 4908 CR Oosterhout (NL); Deilmann, Michael, 45257 Essen (DE); Vogt, Michael, 44797 Bochum (DE); Musch, Thomas, 44879 Bochum (DE); Neuburger, Stephan, 55271 Stadeckenheim (DE)
(74) Vertreter: Gesthuysen Patent- und Rechtsanwälte

(56) Entgegenhaltungen:
- EP-A1- 1 588 900
- WO-A1-80/01513
- WO-A1-2015/090772
- DE-U1-202005 009 609
- US-A1- 2009 007 625
- US-B1- 7 870 793

## Beschreibung

Die vorliegende Erfindung betrifft einen Messstab mit einer Längsachse zur Einführung in den Strömungsquerschnitt eines Rohres und für den Nachweis eines strömenden Mediums in diesem Rohr mit den Merkmalen des Oberbegriffs des Patentanspruchs 1 und eine dafür vorgesehene Messanordnung.

Die Erfindung betrifft insbesondere einen Messstab und eine Messanordnung für den Nachweis eines schnell strömenden Mediums wie beispielsweise Fackelgas. Fackelgase sind Abgase, die bei der Förderung von Erdöl, in Raffinerien sowie in chemischen Betrieben anfallen. Sie enthalten flüchtige organische Verbindungen (VOCs), Schwefeldioxid, Methan und/oder weitere gesundheits- und umweltschädliche Gase. Damit diese nicht unbehandelt in die Umwelt gelangen, werden Fackelgase zuvor verbrannt. Mit deren Verbrennung wird die Umweltbelastung durch diese Gase verringert. Dazu wird das Fackelgas durch Überdruck- und Sicherheitsventile in einen Schornstein geleitet, an dessen Ausgang eine Fackel brennt.

Insofern ist es insbesondere für die Erdöl- bzw. die chemische Industrie von Interesse das Vorhandensein von Fackelgasen in Rohrleitungen nachzuweisen, um beispielsweise deren Verbrennung kontrolliert ablaufen zu lassen.

Das Fackelgas erreicht den Schornstein unter hohem Druck, sodass es eine Strömungsgeschwindigkeit von bis zu 200 m/s aufweisen kann. Diese Geschwindigkeit sowie der hohe Verschmutzungsgrad des Gases stellen hohe Anforderungen an die Methoden für den Nachweis von Fackelgasen.

Aus dem Stand der Technik ist es bekannt, zumindest teilweise gasförmige Medien durch die Wechselwirkung dieser Medien mit Ultraschallsignalen zu detektieren.

Ein bekanntes Messverfahren ist das Laufzeitdifferenzverfahren. Dabei wird ausgenutzt, dass Ultraschallsignale, welche in Bewegungsrichtung eines strömenden Mediums ausgesendet werden, schneller übertragen werden, als Ultraschallsignale, die sich entgegen der Bewegungsrichtung bewegen. Aus dem Laufzeitunterschied zweier Signale, welche die gleiche Strecke zurücklegen, wobei ein Ultraschallsignal in Strömungsrichtung und eines entgegengesetzt dazu ausgesendet werden, lässt sich die Strömungsgeschwindigkeit des zu untersuchenden Gases berechnen. Entsprechende Laufzeitdifferenzmessungen mittels Ultraschallsignalen eignen sich für Strömungsgeschwindigkeiten im Bereich von bis zu 40 m/s.

Die Druckschrift DE 3504622 C1 offenbart eine Vorrichtung zur Messung der Geschwindigkeit eines strömenden Mediums, wobei zwei Sensoren versetzt zueinander an einer Rohrleitung angeordnet sind und wobei die Sensoren eine Inhomogenität des Medium zeitlich nacheinander detektierten. Aus der Kreuzkorrelation der beiden Messsignale wird die Geschwindigkeit des Mediums bestimmt.

Die Druckschrift WO 2015/090772 A1 betrifft eine Messordnung umfassend einen Messstab und zwei Ultraschallwandler, welche einen Signalpfad definieren, wobei der Signalpfad parallel zur oder auf der Längsachse des Messstabs verläuft. Mit Hilfe dieser Messanordnung wird basierend auf dem Laufzeitverfahren die Geschwindigkeit des durch ein Messrohr fließenden Mediums bestimmt.

Ein gattungsgemäßer Messstab ist auch den Druckschriften EP 2 588 900 A1, US 7,870,793 B1, DE 20 2005 009 609 U1 und WO 80/01513 zu entnehmen.

Daneben ist es bekannt, Gase, insbesondere mehrphasige Gase, durch die Wechselwirkung mit elektromagnetischen Wellen, vorzugsweise mit Mikrowellen, zu charakterisieren. Bekannte Verfahren detektieren dabei eine Veränderung der Amplitude und der Phase von Mikrowellensignalen nach dem Durchgang durch das Medium. Dieser Effekt beruht darauf, dass das Medium insbesondere aufgrund der festen und/oder flüssigen Partikel eine von Luft verschiedene Permittivität aufweist, wodurch die von der Permittivität abhängigen Größen, Amplitude und Phase des Mikrowellensignals, durch die Wechselwirkung mit dem Medium verändert werden. Insbesondere bei Rohrleitungen mit großen Nennweiten gestaltet sich der Nachweis mittels Mikrowellensignalen jedoch als schwierig, da Störreflexionen das gemessene Signal überlagern und den Nachweis erschweren.

Die Druckschrift DE 10 2011 102 991 A1 offenbart eine Vorrichtung zur Bestimmung des Volumenanteils einer Komponente eines mehrphasigen Mediums basierend auf der Laufzeit eines elektromagnetischen Signals durch das Medium. Dabei wird der Effekt ausgenutzt, dass die Ausbreitungsgeschwindigkeit des elektromagnatischen Signals materialabhängig ist. Um das relevante Nutzsignal von nicht interessierenden Störsignalen unterscheidbar zu machen, wird das Nutzsignal hinsichtlich seiner Polarisation gekennzeichnet.

Der vorliegenden Erfindung liegt ausgehend von dem zuvor dargelegten Stand der Technik die Aufgabe zugrunde, eine Vorrichtung sowie eine Messanordnung für den Nachweis eines schnell strömenden Mediums anzugeben, die besonders flexibel eingesetzt werden können.

Diese Aufgabe wird gemäß einer ersten Lehre der Erfindung durch einen eingangs genannten Messstab dadurch gelöst, dass der Messstab wenigstens eine erste Sendeeinheit für die Aussendung eines ersten akustischen oder elektromagnetischen Messsignals und wenigstens eine erste Empfangseinheit für den Empfang des ersten Messsignals umfasst, wobei die erste Sendeeinheit und die erste Empfangseinheit eine Messstrecke definieren und wobei die erste Sendeeinheit derart angeordnet ist, dass das erste Messsignal die Messstrecke durchläuft und wobei die erste Empfangseinheit derart angeordnet ist, dass sie zumindest im strömungsfreien Betrieb das erste Messsignal nach dem Durchlauf der Messstrecke empfängt, wobei der Messstab eine entlang seiner Längsachse erstreckte Ausnehmung aufweist, wobei durch die Ausnehmung in dem Messstab wenigstens eine erste Längsfläche und zwei Stirnflächen gebildet werden und in der Ausnehmung zwischen den beiden Stirnflächen die Messstrecke ausgebildet ist,
und wobei durch die Ausnehmung in dem Messstab zusätzlich eine zweite Längsfläche ausgebildet ist, wobei die erste Längsfläche und die zweite Längsfläche an einer Längskante aufeinandertreffen und einen Winkel einschließen und wobei im Betrieb die zweite Längsfläche in Strömungsrichtung vor der Messstrecke angeordnet ist.

Der erfindungsgemäße Messstab stellt eine Vorrichtung für den Nachweis eines schnell strömenden Mediums bereit, wobei die Wechselwirkung des Mediums mit einem akustischen oder elektromagnetischen Messsignal Grundlage für den Nachweis des Mediums ist. Um ideale Bedingungen für diesen Nachweis zu schaffen, wird durch den Messstab, im Detail durch die Anordnung der Sende- und der Empfangseinheit eine Messstrecke definiert. Dabei ist der Messstab derart ausgestaltet, dass im Betrieb das nachzuweisende Medium die Messstrecke durchströmt und innerhalb der Messstrecke mit dem Messsignal wechselwirkt. Insofern ist, insbesondere wenn der Messstab in Rohren mit großen Nennweiten eingesetzt wird, eine definierte Messstrecke gegeben, die vorzugsweise derart ausgestaltet ist, dass das Messsignal überlagernde Störreflexionen weitestgehend vermieden werden. Der erfindungsgemäße Messstab kann damit besonders flexibel eingesetzt werden. Ein Vorteil der mit dem Messstab in einer Einheit realisierten Messstrecke besteht darin, dass die Messstrecke in einem vorgegebenen Rohr sehr einfach installiert werden kann, da der Messstab quasi einfach in den Strömungsquerschnitt von außen in das interessierende Rohr eingeführt werden und dort befestigt werden muss. Die Realisierung der Messstrecke auf oder in dem Messstab gewährleistet, dass die Messstrecke praktisch mit allen Möglichkeiten und der Präzision von modernen industriellen Produktionstechniken gefertigt werden kann, die Güte des Aufbaus der Messstrecke hängt nicht von den industriellen Umgebungs- und Installationsbedingungen des späteren Installationsortes ab.

Grundsätzlich können die erste Sende- und die erste Empfangseinheit einander gegenüberliegend angeordnet sein, sie können alternativ aber auch auf der gleichen Seite des Messstabs angeordnet sein, insbesondere kann die Sendeeinheit derart ausgestaltet sein, dass sie gleichzeitig als Empfangseinheit dient.

Der Nachweis des schnell strömenden Mediums erfolgt auf Grundlage der Wechselwirkung des nachzuweisenden Mediums mit dem ersten Messsignal innerhalb der Messstrecke. Beispielsweise bewirkt die Wechselwirkung eines Mediums mit einem elektromagnetischen Messsignal, beispielsweise mit einem Radar- oder Mikrowellensignal, aufgrund der von Luft verschiedenen Permittivität des Mediums eine Phasenverschiebung und eine Dämpfung der Amplitude des Messsignals. Ist das Messsignal alternativ als akustisches, insbesondere als Ultraschallsignal ausgestaltet, lässt sich ein schnell strömendes Medium dadurch nachweisen, dass die erste Empfangseinheit im strömungsfreien Betrieb ein Ultraschallsignal empfängt, wohingegen bei Anwesenheit eines schnell strömenden Mediums das Messsignal aufgrund der hohen Geschwindigkeit des Mediums sowie der Ankopplung des Ultraschallsignals an das Medium als Träger die Empfangseinheit nicht erreicht. Das von der Empfangseinheit detektierte Messsignal bricht insofern bei der Anwesenheit eines schnell strömenden Mediums ab.

Vorzugsweise ist wenigstens eine weitere Empfangseinheit vorhanden, welche in Strömungsrichtung hinter der ersten Empfangseinheit angeordnet ist, und welche derart ausgestaltet und angeordnet ist, dass sie ein Messsignal empfängt, sofern das Messsignal als Ultraschallsignal ausgestaltet ist und sofern innerhalb der Messstrecke ein schnell strömendes Medium vorhanden ist.

Gemäß einer weiteren bevorzugten Ausgestaltung des erfindungsgemäßen Messstabs sind die erste Sendeeinheit und die erste Empfangseinheit in Bezug auf die Längsachse voneinander beabstandet angeordnet. Die durch die Sendeeinheit und die Empfangseinheit definierte Messstrecke ist dann besonders einfach ausgestaltet. Insbesondere das Auftreten von Störreflexionen wird vermieden. Durch diese Anordnung wird auch eine möglichst lange Messstrecke definiert.

Vorzugsweise verläuft eine elektrische Kontaktierung der ersten Sendeeinheit und der ersten Empfangseinheit innerhalb des Messstabs. Durch die in den Messstab integrierte Kontaktierung weist dieser eine besonders einfache Handhabung, insbesondere in Bezug auf den Einbau in ein Rohr auf, es werden freie Kabelführungen in dem - oft rauen - technischen Prozess vermieden.

In den Messstab selbst werden in einer bevorzugten Ausgestaltung weitere Sensoren integriert, insbesondere solche, die keinen direkte Kontakt mit dem Außenraum des Messstabs kommen müssen. Dies sind beispielsweise Beschleunigungssensoren, mit denen auch geringe Kraftwirkungen und dadurch bedingte Erschütterungen des Messstabs erfasst werden können.

Gemäß einer weiteren Ausgestaltung des Messstabs sind die Sendeeinheit und die Empfangseinheit auf den Umfang des Messstabs aufgesetzt. Der Aufbau sowie die Herstellung eines derart ausgestalteten Messstabs sind besonders einfach.

Erfindungsgemäß weist der Messstab eine entlang seiner Längsachse erstreckte Ausnehmung auf, wobei durch die Ausnehmung in dem Messstab wenigstens eine erste Längsfläche und zwei Stirnflächen gebildet werden und in der Ausnehmung zwischen den beiden Stirnflächen die Messstrecke ausgebildet ist. Dabei ist die erste Längsfläche vorzugsweise eben ausgestaltet. Sie verläuft vorzugsweise im Wesentlichen parallel zur Längsachse, d. h. der Normalenvektor steht im Wesentlichen senkrecht auf der Längsachse. Die Stirnflächen sind vorzugsweise im Wesentlichen senkrecht zur Längsachse angeordnet, d. h. der Normalenvektor der Stirnflächen verläuft parallel zur Längsachse. Eine derartige Ausgestaltung des erfindungsgemäßen Messstabs weist den Vorteil auf, dass durch die ebene Längsfläche die nachzuweisende Strömung im Bereich der Messstrecke hinsichtlich einer Gleichrichtung positiv beeinflusst wird, was sich vorteilhaft auf den Nachweis des Mediums auswirkt, wie weiter unten genauer dargelegt ist.

Erfindungsgemäß ist zusätzlich eine zweite Längsfläche ausgebildet, wobei die erste Längsfläche und die zweite Längsfläche an einer Längskante aufeinandertreffen und einen Winkel einschließen. Die zweite Längsfläche ist dabei im Betrieb des Messstabs in Strömungsrichtung vor der Messstrecke angeordnet. Wird der Messstab von dem nachzuweisenden Medium umströmt, erzeugt die zweite Längsfläche eine Wirbelsignatur, welche durch die Wechselwirkung mit dem Messsignal detektiert werden kann, wie nachfolgend näher erläutert wird.

Weiterhin ist es bevorzugt, wenn durch die Ausnehmung in dem Messstab zusätzlich eine dritte Längsfläche ausgebildet ist, insbesondere wobei die erste Längsfläche, die zweite Längsfläche und die dritte Längsfläche eine Nut in dem Messstab bilden. Bei dieser Variante sind der erste Sender und der erste Empfänger besonders geschützt untergebracht.

Besonders bevorzugt ist wenigstens ein Strömungsgleichrichter zur Erzeugung einer gleichgerichteten Strömung des nachzuweisenden Mediums im Bereich der Messstrecke vorhanden. Eine gleichgerichtete Strömung ist für den Nachweis eines strömenden Mediums insofern vorteilhaft, als dass Schwankungen des gemessenen Effektes aufgrund von zufälligen Turbulenzen der nachzuweisenden Strömung weitestgehend vermieden werden.

Gemäß einer weiteren bevorzugten Ausgestaltung ist der Strömungsgleichrichter in Form von mindestens zwei flügelartigen Profilkörpern ausgestaltet, wobei die flügelartigen Profilkörper zumindest im Bereich der Messstrecke angeordnet sind. Flügelartige Profilkörper sind stromlinienförmig und erzeugen eine besonders gleichmäßige Strömung. Alternativ oder zusätzlich kann der Strömungsgleichrichter auch in Form eines Gitters, das in Strömungsrichtung vor der Messstrecke angeordnet ist, ausgestaltet sein.

Gemäß einer ebenfalls bevorzugten Ausgestaltung des erfindungsgemäßen Messstabs ist wenigstens ein Störkörper vorhanden, wobei der Störkörper in Strömungsrichtung vor der Messstrecke angeordnet ist. Durch den Einsatz eines Störkörpers kann eine gezielte Markierung des strömenden Mediums erfolgen, welche sich für dessen Nachweis vorteilhaft nutzen lässt. Im Detail bilden sich hinter dem Störkörper Wirbel innerhalb der Messstrecke, die mittels des Messsignals detektiert werden können. Dabei erzeugt insbesondere die Kombination eines Strömungsgleichrichters mit einem Störkörper eine Wirbelsignatur, welche einen besonders sicheren Nachweis des strömenden Mediums ermöglicht. Besonders vorteilhaft ist es, wenn der Störkörper im Bereich der im Betrieb gleichgerichteten Strömung angeordnet ist. Dann ist die Wirbelsignatur einer gleichgerichteten Strömung überlagert, sodass die aufgrund des Störkörpers entstehende Wirbelsignatur besonders ausgeprägt ist. Der Störkörper ist vorzugsweise dreieckig ausgestaltet, kann aber auch eine andere Form, beispielsweise eine Viereckform aufweisen.

In einer weiteren Ausgestaltung ist der Störkörper als aktiver Störkörper ausgestaltet, in dem Sinne, dass der Störkörper aktiv Bewegungen ausführen kann, um dem vorbeiströmenden Medium ganz gezielt eine bestimmte Wirbelsignatur aufzuprägen.

Der Nachweis der Wirbelsignatur beruht auf dem Effekt, dass innerhalb des Wirbels Druckschwankungen vorhanden sind, die sich auf die Permittivität auswirken und insofern beispielsweise eine Phasenveränderung des elektromagnetischen Messsignals bewirken. Demnach kann der Effekt der Wechselwirkung zwischen dem Medium und dem Messsignal durch das Vorhandensein der Wirbelsignatur deutlich verstärkt werden.

Zudem kann die Wirbelsignatur ebenfalls mittels akustischer Ultraschallsignale detektiert werden, da die innerhalb des Wirbels herrschenden Druck- bzw. Geschwindigkeitsschwankungen die Phasenlage des akustischen Messsignals verändern, sodass in der Folge ebenfalls ein phasenmoduliertes Messsignal entsteht.

Gemäß einer weiteren bevorzugten Ausgestaltung ist wenigstens ein Drucksensor, vorzugsweise ein Piezowandler, vorhanden, welcher innerhalb der Messstrecke oder in Strömungsrichtung hinter der Messstrecke angeordnet ist. Der Piezowandler ist jedenfalls dem Außenraum des Messstabs ausgesetzt, also auch dem zu detektierenden strömenden Medium, wenn der Messstab in einem Rohr einsatzfähig montiert ist. Der Drucksensor misst im Betrieb den Druck bzw. eventuell auftretende Druckdifferenzen, welche insbesondere dann vorhanden sind, wenn das nachzuweisende Medium im Bereich der Messstrecke eine Wirbelsignatur aufweist. Ist dazu ein Störkörper vorhanden, so ist es ebenfalls bevorzugt, wenn ein Drucksensor alternativ oder zusätzlich im Störkörper angeordnet ist.

Vorzugsweise ist ein Schutzring zwischen dem rohrseitigen Ende und der Messstrecke des Messstabs angeordnet, wobei der Schutzring den Umfang des Messstabs ringförmig umgibt und wobei der Schutzring einen Übertritt von Flüssigkeit von dem rohrseitigen Ende zur Messstrecke verhindert. Wird mithilfe des erfindungsgemäßen Messstabs ein Medium nachgewiesen, welches zumindest teilweise eine Flüssigkeit aufweist, schützt der Schutzring die Messstrecke im Betrieb vor an den Rohrinnenwänden ablaufender Flüssigkeit.

Gemäß einer weiteren bevorzugten Ausgestaltung des erfindungsgemäßen Messstabs ist im Bereich der Ausnehmung wenigstens ein Keramikfenster zur Einkopplung zumindest des ersten Messsignals angeordnet. Gemäß dieser Ausgestaltung ist die Einkopplung des Messsignals in die Messstrecke besonders effizient. Besonders bevorzugt ist das Keramikfenster unmittelbar vor der ersten Sendeeinheit angeordnet.

Vorzugsweise ist ein weiteres Keramikfenster zur Auskopplung des ersten Messsignals vorgesehen, wobei das zweite Keramikfenster unmittelbar vor der Empfangseinheit angeordnet ist. Die Übertragung des Messsignals auf die Empfangseinheit ist gemäß dieser Ausgestaltung besonders effizient.

Darüber hinaus ist es ebenfalls bevorzugt, wenn der Messstab im Bereich der Ausnehmung wenigstens einen Reflektor aufweist, welcher derart angeordnet ist, dass zumindest das erste Messsignal nach dem Durchlauf durch die Messstrecke in Richtung auf die Empfangseinheit reflektiert wird. Vorzugsweise sind in dieser Anordnung die erste Sendeeinheit und die erste Empfangseinheit auf derselben Seite der Messstrecke angeordnet. Besonders bevorzugt ist die erste Sendeeinheit derart ausgestaltet, dass sie gleichzeitig als erste Empfangseinheit dient. Diese Anordnung weist den Vorteil auf, dass die Wechselwirkungsstrecke zwischen dem nachzuweisenden Medium und dem Messsignal verlängert ist, sodass der nachzuweisende Effekt ebenfalls verstärkt wird.

Diese Ausgestaltung kann dadurch weiter verbessert werden, dass die Messstrecke einen Resonator für das Messsignal bildet. Wird mittels des Messsignals, welches gemäß dieser Ausgestaltung besonders bevorzugt in Form eines Mikrowellensignals vorliegt, eine Resonatormode angeregt, kann die Wechselwirkungsstrecke zwischen dem nachzuweisenden Medium und dem Messsignal um ein vielfaches erhöht werden, wodurch der nachzuweisende Effekt deutlich verstärkt werden kann.

Gemäß einer nächsten Ausgestaltung des erfindungsgemäßen Messstabs sind wenigstens eine zweite Sendeeinheit für die Aussendung eines zweiten akustischen oder elektromagnetischen Messsignals und wenigstens eine zweite Empfangseinheit für den Empfang des zweiten Messsignals vorhanden, wobei die zweite Sendeeinheit derart angeordnet ist, dass das zweite Messsignal die Messstrecke durchläuft, wobei die zweite Empfangseinheit derart angeordnet ist, dass sie zumindest im strömungsfreien Betrieb das zweite Messsignal nach dem Durchlauf der Messstrecke empfängt und wobei die zweite Sendeeinheit und die zweite Empfangseinheit in Strömungsrichtung hinter der ersten Sendeeinheit und in Strömungsrichtung hinter der ersten Empfangseinheit angeordnet sind. In vorteilhafter Weise kann das Medium gemäß dieser Ausgestaltung an zwei Positionen vermessen werden. Wird im Betrieb eine Markierung, beispielsweise in Form eines Wirbels, in die Strömung eingebracht, so kann nach dem Kreuzkorrelationsverfahren neben dem Nachweis des Vorhandenseins von dem strömenden Medium ebenfalls eine Information über die Laufzeit entsprechender Markierungen und insofern über die Geschwindigkeit des Mediums ermittelt werden.

Gemäß einer Ausgestaltung des erfindungsgemäßen Messstabs ist wenigstens eine Auswerteeinheit vorhanden, welche zumindest mit der ersten Empfangseinheit und/oder der zweiten Empfangseinheit verbunden ist. Im Betrieb leitet die erste Empfangseinheit und/oder die zweite Empfangseinheit das empfangene Messsignal an die Auswerteeinheit weiter, die anschließend das Messsignal hinsichtlich der Wechselwirkungsparameter auswertet. Die Auswerteeinheit bestimmt die Amplitude und/oder die Phase und/oder die Intensität und/oder Laufzeit des Messsignals und/oder die Kreuzkorrelation des ersten und des zweiten Messsignals. Die Auswerteeinheit liefert jedenfalls einen aus dem ersten und/oder zweiten Messsignal erhaltenen Messwert.

In den Messstab integriert ist vorzugsweise eine Übertragungsvorrichtung, die den Messwert in ein geeignetes elektrisches Signal codiert und nach außerhalb des Messstabes ausgibt, beispielsweise über eine elektrische Kontaktierungsvorrichtung. Die Codierung kann gemäß einem oder auch gemäß mehrerer Schnittstellenstandards erfolgen. In einer Ausführungsform ist der Messstab als Zweileitergerät mit einer 4 mA bis 20 mA-Schnittstelle ausgestaltet. In einer weiteren Ausgestaltung ist alternativ oder zusätzlich ein digitales Übertragungsprotokoll vorgesehen, beispielsweise in Form einer HART-Schnittstelle.

Gemäß einer zweiten Lehre der vorliegenden Erfindung ist die eingangs dargelegte Aufgabe gelöst durch eine Messanordnung umfassend ein Rohr und einen Messstab gemäß einer der zuvor beschriebenen Ausgestaltungen, wobei der Messstab zumindest teilweise innerhalb des Rohres angeordnet ist. Die erfindungsgemäße Messanordnung weist den Vorteil auf, dass unabhängig von der Nennweite des Rohres eine definierte Messstrecke vorhanden ist, welche ideale Bedingung für den Nachweis des Mediums schafft. Eine Beeinflussung des Nachweises beispielsweise aufgrund von Störreflexionen kann durch die erfindungsgemäße Messanordnung vermieden werden.

Gemäß einer bevorzugten Ausgestaltung der erfindungsgemäßen Messanordnung weist das Rohr einen Radius auf, wobei der Messstab höchstens um die Hälfte des Radius, vorzugsweise höchstens um ein Drittel des Radius in das Rohr hineinragt.

Besonders bevorzugt ist eine erfindungsgemäße Messanordnung, wenn ein Störkörper vorhanden ist, wobei der Störkörper in Strömungsrichtung vor dem Messstab angeordnet ist. Insbesondere wenn gleichzeitig wenigstens ein Strömungsgleichrichter vorhanden ist, kann ein besonders sicherer Nachweis des strömenden Mediums erfolgen.

Im Einzelnen gibt es nun eine Vielzahl von Möglichkeiten, den erfindungsgemäßen Messstab und die erfindungsgemäße Messanordnung auszugestalten. Dazu wird verwiesen sowohl auf die den unabhängigen Patentansprüchen nachgeordneten Patentansprüche als auch auf die nachfolgende Beschreibung von bevorzugten Ausführungsbeispielen in Verbindung mit der Zeichnung. In der Zeichnung zeigt
- Fig. 1: ein erstes nicht-erfindungsgemäßes Ausführungsbeispiel eines Messstabs in perspektivischer Ansicht mit aufgesetzter Sende- und Empfangseinheit,
- Fig. 2: ein zweites nicht-erfindungsgemäßes Ausführungsbeispiel eines Messstabs in perspektivischer Ansicht mit einer die Messstrecke des Messstabs definierenden Ausnehmung,
- Fig. 3: ein drittes Ausführungsbeispiel eines erfindungsgemäßen Messstabs in perspektivischer Ansicht mit einer die Messstrecke des Messstabs definierenden Ausnehmung mit integrierter Verwirbelungskante,
- Fig. 4: eine Schnittdarstellung eines vierten Ausführungsbeispiels eines erfindungsgemäßen Messstabs mit einer Ausnehmung,
- Fig. 5: eine Schnittdarstellung eines fünften Ausführungsbeispiels eines erfindungsgemäßen Messstabs mit einer Ausnehmung und mit Strömungsgleichrichtern,
- Fig. 6: eine Schnittdarstellung eines sechsten Ausführungsbeispiels eines erfindungsgemäßen Messstabs mit einem Schutzring,
- Fig. 7: eine Schnittdarstellung eines siebten Ausführungsbeispiels eines erfindungsgemäßen Messstabs mit einem Störkörper,
- Fig. 8: eine Schnittdarstellung eines achten Ausführungsbeispiels eines erfindungsgemäßen Messstabs,
- Fig. 9: eine Schnittdarstellung eines neunten Ausführungsbeispiels eines erfindungsgemäßen Messstabs mit einem Reflektor in der Messstrecke,
- Fig. 10: eine Schnittdarstellung eines zehnten Ausführungsbeispiels eines erfindungsgemäßen Messstabs mit einer doppelt ausgeführten Messstrecke,
- Fig. 11: eine Schnittdarstellung eines ersten Ausführungsbeispiels einer erfindungsgemäßen Messanordnung,

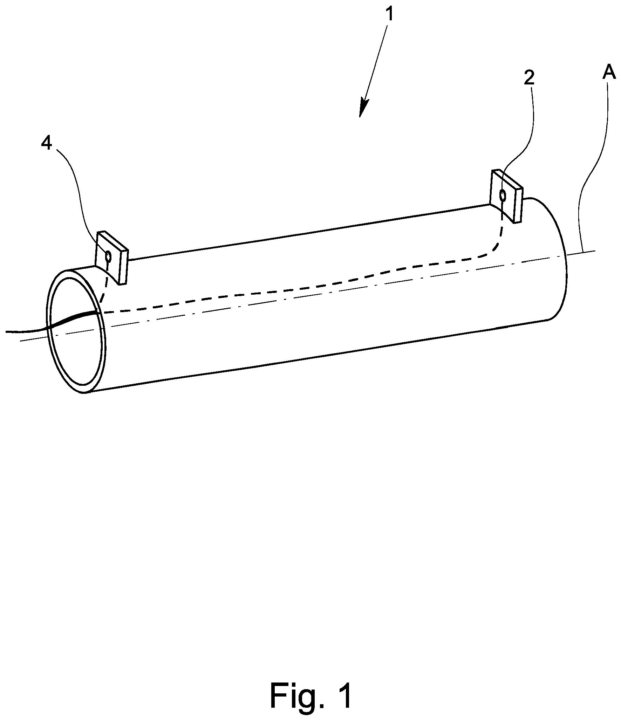

In Fig. 1 ist dargestellt ein erstes nicht-erfindungsgemäßes Ausführungsbeispiel eines Messstabs 1 mit einer Längsachse A zur Einführung in den Strömungsquerschnitt eines Rohres und für den Nachweis eines strömenden Mediums in diesem Rohr in dreidimensionaler Ansicht. Der Messstab 1 weist eine erste Sendeeinheit 2 für die Aussendung eines ersten akustischen oder elektromagnetischen Messsignals und eine erste Empfangseinheit 4 für den Empfang des ersten Messsignals 3 auf, wobei die erste Sendeeinheit 2 und die erste Empfangseinheit 4 in Bezug auf die Längsachse A beabstandet voneinander angeordnet sind. Die Sendeeinheit 2 und die Empfangseinheit 4 definieren eine Messstrecke, welche im Betrieb von dem nachzuweisendem Medium durchströmt wird. Dabei ist die erste Sendeeinheit 2 derart angeordnet, dass das erste Messsignal die Messstrecke durchläuft und die erste Empfangseinheit 4 ist derart angeordnet, dass sie zumindest im strömungsfreien Betrieb das erste Messsignal nach dem Durchlauf der Messstrecke empfängt. Im dargestellten Ausführungsbeispiel sind die Sendeeinheit 2 und die Empfangseinheit 4 auf den Umfang des Messstabs 1 aufgesetzt. Zudem ist eine elektrische Kontaktierung der Sende- 2 und der Empfangseinheit 4 vorhanden, die innerhalb des Messstabs 1 verläuft.

Das dargestellte erste Ausführungsbeispiel zeigt insofern einen Messstab 1 für den Nachweis eines strömenden Mediums in einem Rohr, welcher aufgrund seiner Ausgestaltung besonders einfach in der Handhabung und in der Herstellung ist und welcher besonders flexibel, insbesondere in Kombination mit Rohren großer Nennweiten eingesetzt werden kann.

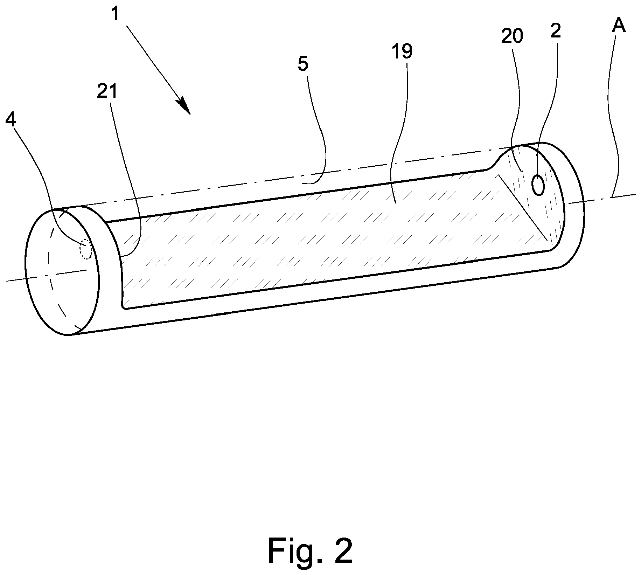

Fig. 2 zeigt ein zweites nicht-erfindungsgemäßes Ausführungsbeispiel eines Messstabs 1 in dreidimensionaler Ansicht, wobei der Messstab 1 eine erste Sendeeinheit 2 für die Aussendung eines ersten akustischen oder elektromagnetischen Messsignals und eine erste Empfangseinheit 4 für den Empfang des ersten Messsignals aufweist, wobei die Sendeeinheit 2 und die Empfangseinheit 4 eine Messstrecke definieren und wobei die Sendeeinheit 2 und die Empfangseinheit 4 derart angeordnet sind, dass das Messsignal die Messstrecke durchläuft und zumindest im strömungsfreien Betrieb nach dem Durchlauf der Messstrecke von der Empfangseinheit 4 empfangen wird. Darüber hinaus weist der Messstab 1 eine entlang der Längsachse A erstreckte Ausnehmung 5 auf, wobei durch die Ausnehmung 5 in dem Messstab wenigstens eine erste ebene Längsfläche 19 und zwei Stirnflächen 20, 21 gebildet werden und in der Ausnehmung 5 zwischen den beiden Stirnflächen 20, 21 die Messstrecke ausgebildet ist.

Eine derartige Ausgestaltung der Messstrecke weist den Vorteil auf, dass zumindest durch die ebene Längsfläche 19 die nachzuweisende Strömung im Bereich der Messstrecke gleichgerichtet wird, wodurch der Nachweis eines Medium aufgrund der Wechselwirkung des Mediums mit dem Messsignal vereinfacht wird.

Im Ergebnis zeigt Fig. 2 ein Ausführungsbeispiel eines Messstabs 1, der besonders flexibel auch bei Rohren großer Nennweite eingesetzt werden kann und der verbesserte Eigenschaften bezüglich des Nachweises eines schnell strömenden Mediums aufweist.

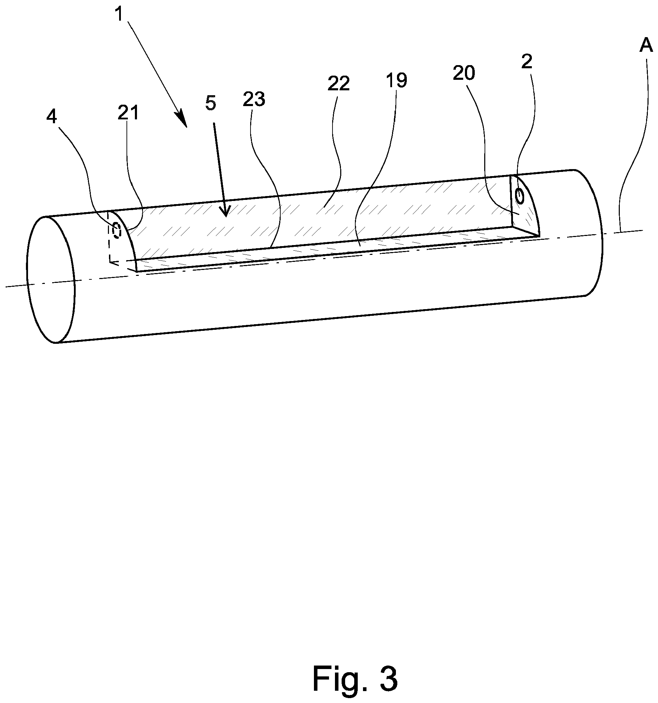

Fig. 3 zeigt ein weiteres drittes Ausführungsbeispiel eines erfindungsgemäßen Messstabs 1 mit einer Längsachse A in dreidimensionaler Ansicht. Der Messstab 1 umfasst eine erste Sendeeinheit 2 und eine erste Empfangseinheit 4, die eine Messstrecke definieren, wobei die erste Sendeeinheit 2 derart angeordnet ist, dass das erste Messsignal die Messstrecke durchläuft und wobei die erste Empfangseinheit 4 derart ist, dass sie zumindest im strömungsfreien Betrieb das erste Messsignal nach dem Durchlauf der Messstrecke empfängt. Darüber hinaus weist der Messstab 1 eine entlang seiner Längsachse A erstreckte Ausnehmung 5 auf, wobei durch die Ausnehmung 5 in dem Messstab 1 eine erste Längsfläche 19 und zwei Stirnflächen 20, 21 gebildet werden und in der Ausnehmung 5 zwischen den beiden Stirnflächen 20, 21 die Messstrecke ausgebildet ist. Zusätzlich ist durch die Ausnehmung 5 eine zweite Längsfläche 22 ausgebildet, wobei die erste Längsfläche 19 und die zweite Längsfläche 22 an einer Längskante 23 aufeinander treffen und einen Winkel einschließen.

Im Betrieb wird der dargestellte Messstab 1 vorzugsweise derart in den Strömungsquerschnitt eines Rohres eingeführt, dass die zweite Längsfläche 22 in Strömungsrichtung vor der Messstrecke angeordnet ist. Dann erzeugt die zweite Längsfläche 22 eine Wirbelsignatur des strömenden Mediums innerhalb der Messstrecke, wodurch der Nachweis des Mediums, wie auch im Rahmen der nachfolgenden Ausführungsbeispiele erläutert, vereinfacht wird.

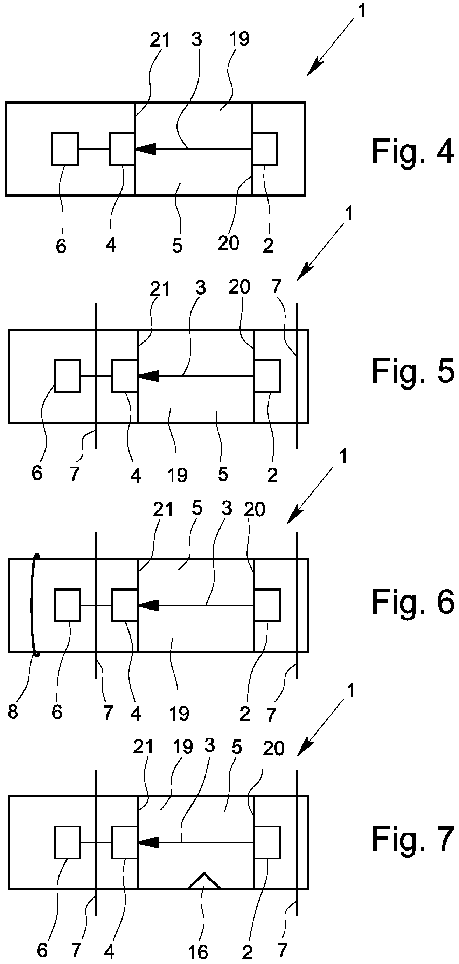

Fig. 4 zeigt eine Schnittdarstellung eines vierten Ausführungsbeispiels eines Messstabs 1 für den Nachweis eines schnell strömenden Mediums in einem Rohr. Der Messstab 1 weist eine erste Sendeeinheit 2 für die Aussendung eines ersten akustischen oder elektromagnetischen Messsignals 3 und eine erste Empfangseinheit 4 für den Empfang des ersten Messsignals 3 auf. Die Sendeeinheit 2 und die Empfangseinheit 4 sind im dargestellten Ausführungsbeispiel in den Messstab 1 integriert. Zudem weist der Messstab 1 eine entlang der Längsachse erstreckte Ausnehmung 5 auf, wobei durch die Ausnehmung 5 eine erste Längsfläche 19 und zwei Stirnflächen 20, 21 gebildet werden, und wobei in der Ausnehmung 5 zwischen den beiden Stirnflächen eine Messstrecke ausgebildet ist. Im Betrieb des Messstabs durchströmt das nachzuweisende Medium die Messstrecke. Die erste Sendeeinheit 2 ist derart angeordnet, dass das erste Messsignal 3 die Messstrecke durchläuft und die erste Empfangseinheit 4 ist derart angeordnet, dass sie zumindest im strömungsfreien Betrieb das erste Messsignal 3 nach dem Durchlauf der Messstrecke empfängt.

Zudem ist eine Auswerteeinheit 6 mit der ersten Empfangseinheit 4 verbunden. Ist innerhalb der Messstrecke ein schnell strömendes Medium vorhanden, so bewirkt die Wechselwirkung des Messsignals mit dem Medium einen messbaren Effekt, welcher von der Empfangseinheit 4 detektiert und von der Auswerteeinheit 6 bestimmt wird. Beispielsweise bewirkt die Wechselwirkung eines Mediums mit einem elektromagnetischen Messsignal 3 des Mediums eine von der Permittivität des Mediums abhängige Phasenverschiebung und/oder eine Dämpfung der Amplitude. Ist das Messsignal 3 alternativ als akustisches, insbesondere als Ultraschallsignal ausgestaltet, lässt sich das Medium beispielsweise dadurch nachweisen, dass die Empfangseinheit 4 im strömungsfreien Betrieb ein Ultraschallsignal empfängt, wohingegen bei Anwesenheit eines schnell strömenden Mediums das Messsignal 3 die Empfangseinheit 4 aufgrund der hohen Geschwindigkeit des Mediums nicht erreicht, wodurch das detektierte Signal abbricht.

Im Ergebnis zeigt Fig. 4 ein vorteilhaftes Ausführungsbeispiel eines Messstabs 1, der einen sicheren Nachweis eines schnell strömenden Mediums bereitstellt und der aufgrund seiner Ausgestaltung besonders einfach in Rohren unterschiedlicher Nennweite eingesetzt werden kann.

Fig. 5 zeigt eine Schnittdarstellung eines fünften Ausführungsbeispiels eines Messstabs 1, umfassend eine erste Sendeeinheit 2 für die Aussendung eines ersten akustischen oder elektromagnetischen Messsignals 3 und eine erste Empfangseinheit 4 für den Empfang des ersten Messsignals 3, wobei die Sendeeinheit 2 und die Empfangseinheit 4 in den Messstab 1 integriert sind. Durch eine Ausnehmung 5 werden eine erste Längsfläche 19 und zwei Stirnflächen 20, 21 gebildet, wobei in der Ausnehmung 5 zwischen den Stirnflächen 20, 21 die Messstrecke ausgebildet ist, durch die im Betrieb das nachzuweisende Medium strömt. Zudem ist eine Auswerteeinheit 6 vorhanden, welche mit der Empfangseinheit 4 verbunden ist und welche ebenfalls in den Messstab 1 integriert ist.

Darüber hinaus weist der dargestellte Messstab 1 einen Strömungsgleichrichter in Form von zwei flügelartigen Profilkörpern 7 auf, welche im Bereich der Messstrecke angeordnet sind, sodass sie im Betrieb eine gleichgerichtete Strömung des nachweisenden Mediums im Bereich der Messstrecke bewirken.

Eine gleichgerichtete Strömung hat den Vorteil, dass die aufgrund der Wechselwirkung des Mediums mit dem Messsignal 3 nachgewiesenen Effekte weniger Schwankungen unterliegen, die auf zufällige Turbulenzen zurückzuführen sind. Der Nachweis eines strömenden Mediums erfolgt gemäß diesem Ausführungsbeispiel mit besonders hoher Zuverlässigkeit.

In Fig. 6 ist eine Schnittdarstellung eines weiteren sechsten Ausführungsbeispiels eines Messstabs 1 dargestellt, umfassend eine erste Sendeeinheit 2 für die Aussendung eines ersten akustischen oder elektromagnetischen Messsignals 3 und eine erste Empfangseinheit 4 für den Empfang des ersten Messsignals 3, wobei die Sendeeinheit 2 und die Empfangseinheit 4 in den Messstab 1 integriert sind. Durch eine Ausnehmung 5 werden eine erste Längsfläche 19 und zwei Stirnflächen 20, 21 gebildet, wobei in der Ausnehmung 5 zwischen den Stirnflächen 20, 21 die Messstrecke ausgebildet ist, durch die im Betrieb das nachzuweisende Medium strömt. Zudem ist eine Auswerteeinheit 6 vorhanden, welche mit der Empfangseinheit 4 verbunden ist und welche ebenfalls in den Messstab 1 integriert ist. Darüber hinaus sind flügelartige Profilkörper 7 im Bereich der Messstrecke angeordnet, welche im Betrieb eine gleichgerichtete Strömung im Bereich der Messstrecke bewirken.

Zudem weist der in Fig. 6 dargestellte Messstab 1 einen Schutzring 8 auf, der zwischen dem rohrseitigen Ende und der Messstrecke des Messstabs 1 angeordnet ist, wobei der Schutzring 8 den Umfang des Messstabs 1 ringförmig umgibt und wobei der Schutzring 8 einen Übertritt von Flüssigkeit von dem rohrseitigen Ende zur Messstrecke verhindert. Ein derartiges Ausführungsbeispiel ist insbesondere dann vorteilhaft, wenn der Messstab 1 für den Nachweis von Medien eingesetzt wird, welche zumindest teilweise eine Flüssigkeit aufweisen. Im Betrieb schützt der Schutzring 8 dann die Messstrecke vor an der Rohrinnenwand ablaufender Flüssigkeit.

In Fig. 7 ist ein weiteres besonders vorteilhaftes Ausführungsbeispiel des erfindungsgemäßen Messstabs 1 in Schnittdarstellung gezeigt. Wie die zuvor beschriebenen Ausführungsbeispiele weist auch der in Fig. 7 dargestellte Messstab 1 eine erste Sendeeinheit 2 für die Aussendung eines ersten akustischen oder elektromagnetischen Messsignals 3 und eine erste Empfangseinheit 4 für den Empfang des ersten Messsignals 3, wobei die Sendeeinheit 2 und die Empfangseinheit 4 in den Messstab 1 integriert sind. Durch eine Ausnehmung 5 werden eine erste Längsfläche 19 und zwei Stirnflächen 20, 21 gebildet, wobei in der Ausnehmung 5 zwischen den Stirnflächen 20, 21 die Messstrecke ausgebildet ist, durch die im Betrieb das nachzuweisende Medium strömt. Ebenfalls ist eine Auswerteeinheit 6 vorhanden, welche mit der Empfangseinheit 4 verbunden ist und welche in den Messstab 1 integriert ist. Darüber hinaus sind flügelartige Profilkörper 7 im Bereich der Messstrecke angeordnet, die im Betrieb eine gleichgerichtete Strömung des Mediums im Bereich der Messstrecke bewirken.

Zudem weist der in Fig. 7 dargestellte Messstab einen Störkörper 16 auf, der in Strömungsrichtung vor der Messstrecke angeordnet ist. Im Betrieb bildet das nachzuweisende Medium hinter dem Störkörper Wirbel innerhalb der Messstrecke, die mittels des Messsignals 3 detektiert werden können. Dabei erzeugt insbesondere die Kombination der flügelartigen Profilkörper 7 mit dem Störkörper 16 eine Wirbelsignatur, die sich mit besonders hoher Zuverlässigkeit detektieren lässt.

In einer weiteren Ausgestaltung ist der Störkörper 16 ein aktiver Störkörper, der durch eine entsprechende Ansteuerung ausgelenkt , insbesondere zu Schwingungen angeregt wird, so dass eine bestimmte Wirbelsignatur gezielt erzeugt wird.

Ist das Messsignal 3 ein elektromagnetisches Signal, beispielsweise ein Radar- oder Mikrowellensignal, beruht der Nachweis der Wirbelsignatur auf dem Effekt, dass innerhalb des Wirbels Druckschwankungen vorhanden sind, die sich auf die Permittivität auswirken und insofern beispielsweise eine Phasenveränderung des Radar- oder Mikrowellensignals bewirken, welche von der Empfangseinheit 4 detektiert und von der Auswerteeinheit 6 bestimmt wird. Demnach kann der nachweisbare Effekt der Wechselwirkung zwischen dem Medium und dem Messsignal durch das Vorhandensein der Wirbelsignatur deutlich verstärkt werden. Dabei tritt die Wirbelsignatur im dargestellten Ausführungsbeispiel besonders deutlich hervor, da der Störkörper 16 innerhalb der flügelartigen Profilkörper 7 angeordnet ist, also im Bereich der im Betrieb erzeugten gleichgerichteten Strömung liegt. Zudem kann die Wirbelsignatur ebenfalls mittels akustischer Ultraschallsignale detektiert werden, da die innerhalb des Wirbels herrschenden Druck- bzw. Geschwindigkeitsschwankungen die Phasenlage des akustischen Messsignals verändern, sodass in der Folge ebenfalls ein phasenmoduliertes Messsignal entsteht.

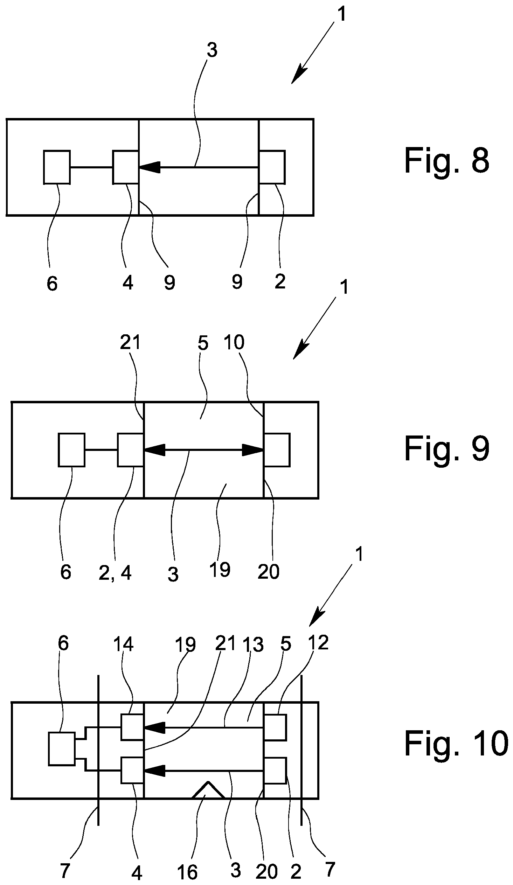

Fig. 8 zeigt eine Schnittdarstellung eines achten Ausführungsbeispiels eines erfindungsgemäßen Messstabs 1, der eine erste Sendeeinheit 2 für die Aussendung eines ersten akustischen oder elektromagnetischen Messsignals 3 und eine erste Empfangseinheit 4 für den Empfang des ersten Messsignals 3 aufweist, wobei die Sendeeinheit 2 und die Empfangseinheit 4 in den Messstab 1 integriert sind. Zudem ist eine Auswerteeinheit 6 vorhanden, die mit der Empfangseinheit 4 verbunden ist und die ebenfalls in den Messstab 1 integriert ist. Zur Einkopplung des Messsignals 3 in die Messstrecke ist zwischen der Sendeeinheit 2 und der Ausnehmung ein Keramikfenster 9 angeordnet. Die Einkopplung des Messsignals erfolgt besonders verlustarm. Ebenso ist zwischen der Ausnehmung 5 und der Empfangseinheit 4 ein Keramikfenster 9 angeordnet, wodurch das Messsignal 3 besonders effizient, d.h. verlustarm ausgekoppelt werden kann.

In Fig. 9 ist ein neuntes Ausführungsbeispiel eines erfindungsgemäßen Messstabs 1 dargestellt, der eine erste Sendeeinheit 2 für die Aussendung eines ersten akustischen oder elektromagnetischen Messsignals 3 und eine erste Empfangseinheit 4 für den Empfang des ersten Messsignals 3 aufweist, wobei die Sendeeinheit 2 und die Empfangseinheit 4 in den Messstab 1 integriert sind. Durch eine Ausnehmung 5 werden eine erste Längsfläche 19 und zwei Stirnflächen 20, 21 gebildet, wobei in der Ausnehmung 5 zwischen den Stirnflächen 20, 21 die Messstrecke ausgebildet ist, durch die im Betrieb das nachzuweisende Medium strömt. Zudem ist eine Auswerteeinheit 6 vorhanden, die mit der Empfangseinheit 4 verbunden ist und die ebenfalls in den Messstab 1 integriert ist. Im Unterschied zu den vorhergehenden Ausführungsbeispielen ist die Sendeeinheit 2 derart ausgestaltet, dass sie gleichzeitig als Empfangseinheit 4 für das Messsignal 3 dient. An der der Sendeeinheit 2 gegenüberliegenden Seite der Messstrecke ist ein Reflektor 10 angeordnet, welcher im Betrieb das erste Messsignal 3 nach dem Durchlauf durch die Messstrecke in Richtung auf die Empfangseinheit 4 reflektiert. Dieses Ausführungsbeispiel weist den Vorteil auf, dass es besonders einfach ausgestaltet ist. Darüber hinaus ist der Effekt der Wechselwirkung des strömenden Mediums mit dem Messsignal 3 aufgrund der längeren Wechselwirkungsstrecke verstärkt, sodass ein besonders sicherer Nachweis des strömenden Mediums möglich ist.

Fig. 10 zeigt eine Schnittdarstellung eines zehnten Ausführungsbeispiels eines erfindungsgemäßen Messstabs 1 umfassend eine erste Sendeeinheit 2 für die Aussendung eines ersten akustischen oder elektromagnetischen Messsignals 3 und eine erste Empfangseinheit 4 für den Empfang des ersten Messsignals 3 aufweist, wobei die Sendeeinheit 2 und die Empfangseinheit 4 in den Messstab 1 integriert sind. Durch eine Ausnehmung 5 werden eine erste Längsfläche 19 und zwei Stirnflächen 20, 21 gebildet, wobei in der Ausnehmung 5 zwischen den Stirnflächen 20, 21 die Messstrecke ausgebildet ist, durch die im Betrieb das nachzuweisende Medium strömt. Flügelartige Profilkörper 7 sind im Bereich der Messstrecke angeordnet und erzeugen im Betrieb ein gleichgerichtete Strömung im Bereich der Messstrecke. Zudem weist der dargestellte Messstab 1 einen Störkörper 16 auf, welcher in Strömungsrichtung vor der Messstrecke angeordnet ist und welcher im Betrieb eine Wirbelsignatur des strömenden Mediums im Bereich der Messstrecke erzeugt.

Darüber hinaus weist der dargestellte Messstab 1 eine zweite Sendeeinheit 12 und eine zweite Empfangseinheit 14 auf, welche in Strömungsrichtung hinter der ersten Sendeeinheit 2 und hinter der ersten Empfangseinheit 4 angeordnet sind. Zudem ist eine Auswerteeinheit 6 vorhanden, die mit der ersten Empfangseinheit 4 und der zweiten Empfangseinheit 14 verbunden ist und die ebenfalls in den Messstab 1 integriert ist. Die von dem Störkörper 16 im Betrieb erzeugte Wirbelsignatur durchläuft sowohl das erste Messsignal 3 als auch das zweite Messsignal 13. Mithilfe des Kreuzkorrelationsverfahrens, mittels dessen die von zwei Empfangseinheiten 4 und 14 gemessenen Messsignale 3 und 13 in Hinblick auf deren Übereinstimmung miteinander verglichen werden, lässt sich neben dem Nachweis des Vorhandenseins eines strömenden Mediums ebenfalls eine Information über dessen Strömungsgeschwindigkeit gewinnen.

Folglich stellt dieses Ausführungsbeispiel einen Messstab 1 bereit, mit welchem ein besonders genauer und effektiver Nachweis von einem schnell strömendem Medium möglich ist.

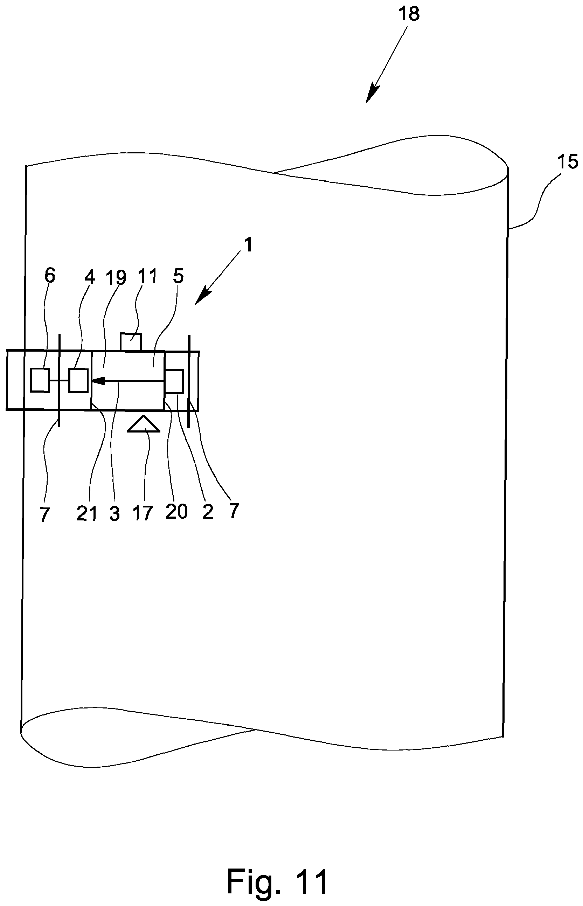

Fig. 11 zeigt eine Schnittdarstellung eines ersten Ausführungsbeispiels einer erfindungsgemäßen Messanordnung 18. Die Messanordnung 18 umfasst ein Rohr 15 und ein Ausführungsbeispiel eines erfindungsgemäßen Messstabs 1, wobei der Messstab 1 an dem Rohr 15 derart angeordnet ist, dass der Messstab 1 um weniger als die Hälfte des Radius in das Rohr 15 hineinragt.

Der Messstab 1 weist eine erste Sendeeinheit 2 für die Aussendung eines ersten akustischen oder elektromagnetischen Messsignals 3 und eine erste Empfangseinheit 4 für den Empfang des ersten Messsignals 3 auf, wobei die Sendeeinheit 2 und die Empfangseinheit 4 in den Messstab 1 integriert sind. Durch eine Ausnehmung 5 werden eine erste Längsfläche 19 und zwei Stirnflächen 20, 21 gebildet, wobei in der Ausnehmung 5 zwischen den Stirnflächen 20, 21 die Messstrecke ausgebildet ist, durch die im Betrieb das nachzuweisende Medium strömt. Zudem ist eine Auswerteeinheit 6 vorhanden, die mit der Empfangseinheit 4 verbunden ist und die ebenfalls in den Messstab 1 integriert ist. Flügelartige Profilkörper 7 sind im Bereich der Messstrecke angeordnet und erzeugen im Betrieb eine gleichgerichtete Strömung im Bereich der Messstrecke.

Vor dem Messstab 1 ist ein Störkörper 17 angeordnet, welcher im Bereich der Messstrecke im Betrieb eine Wirbelsignatur des nachzuweisenden Mediums erzeugt, wodurch der Nachweis eines schnell strömenden Mediums deutlich verbessert werden kann.

Darüber hinaus weist der Messstab 1 einen Drucksensor 11 auf, welcher in Strömungsrichtung hinter der Messstrecke angeordnet ist und welcher im Betrieb die durch die Wirbelsignatur erzeugten Druckschwankungen des nachzuweisenden Mediums detektiert.

Im Ergebnis stellt das dargestellte Ausführungsbeispiel eine Messanordnung 18 bereit, die sich besonders gut für den Nachweis eines schnell strömenden Mediums eignet.

### Bezugszeichenliste

- 1: Messstab
- 2: Erste Sendeeinheit
- 3: Erstes Messsignal
- 4: Erste Empfangseinheit
- 5: Ausnehmung
- 6: Auswerteeinheit
- 7: Flügelartiger Profilkörper
- 8: Schutzring
- 9: Keramikfenster
- 10: Reflektor
- 11: Drucksensor
- 12: Zweite Sendeeinheit
- 13: Zweites Messsignal
- 14: Zweite Empfangseinheit
- 15: Rohr
- 16: Störkörper
- 17: Zweiter Störkörper
- 18: Messanordnung
- 19: erste Längsfläche
- 20: Stirnfläche
- 21: Stirnfläche
- 22: zweite Längsfläche
- 23: Längskante

## Patentansprüche

1. Messstab (1) mit einer Längsachse (A) zur Einführung in den Strömungsquerschnitt eines Rohres und für den Nachweis eines strömenden Mediums in diesem Rohr, umfassend wenigstens eine erste Sendeeinheit (2) für die Aussendung eines ersten akustischen oder elektromagnetischen Messsignals (3) und wenigstens eine erste Empfangseinheit (4) für den Empfang des ersten Messsignals, wobei die erste Sendeeinheit (2) und die erste Empfangseinheit (4) eine Messstrecke definieren, wobei die erste Sendeeinheit (2) derart angeordnet ist, dass das erste Messsignal (5) die Messstrecke durchläuft und wobei die erste Empfangseinheit (4) derart angeordnet ist, dass sie zumindest im strömungsfreien Betrieb das erste Messsignal (3) nach dem Durchlauf der Messstrecke empfängt,
wobei der Messstab (1) eine entlang seiner Längsachse (A) erstreckte Ausnehmung (5) aufweist,
**dadurch gekennzeichnet,**
**dass** durch die Ausnehmung (5) in dem Messstab (1) wenigstens eine erste Längsfläche (19) und zwei Stirnflächen (20, 21) gebildet werden und in der Ausnehmung (5) zwischen den beiden Stirnflächen (20, 21) die Messstrecke ausgebildet ist,
**dass** durch die Ausnehmung (5) in dem Messstab (1) zusätzlich eine zweite Längsfläche (22) ausgebildet ist, wobei die erste Längsfläche (19) und die zweite Längsfläche (22) an einer Längskante (23) aufeinandertreffen und einen Winkel einschließen und wobei die zweite Längsfläche im Betrieb des Messstabs in Strömungsrichtung vor der Messstrecke angeordnet ist.

2. Messstab (1) nach Anspruch 1, **dadurch gekennzeichnet, dass** die erste Sendeeinheit (2) und die erste Empfangseinheit (4) in Bezug auf die Längsachse (A) beabstandet voneinander angeordnet sind.

3. Messstab (1) nach Anspruch 1 oder 2, **dadurch gekennzeichnet, dass** eine elektrische Kontaktierung der ersten Sendeeinheit (2) und der ersten Empfangseinheit (4) innerhalb des Messstabs (1) verläuft.

4. Messstab (1) nach einem der Ansprüche 1 bis 3, **dadurch gekennzeichnet, dass** durch die Ausnehmung (5) in dem Messstab (1) zusätzlich eine dritte Längsfläche ausgebildet ist, insbesondere wobei die erste Längsfläche (19), die zweite Längsfläche (22) und die dritte Längsfläche eine Nut in dem Messstab (1) bilden.

5. Messstab (1) nach einem der Ansprüche 1 bis 4, **dadurch gekennzeichnet, dass** wenigstens ein Strömungsgleichrichter zur Erzeugung einer gleichgerichteten Strömung des nachzuweisenden Mediums im Bereich der Messstrecke vorhanden ist.

6. Messstab (1) nach Anspruch 5, **dadurch gekennzeichnet, dass** der Strömungsgleichrichter in Form von mindestens zwei flügelartigen Profilkörpern (7) ausgebildet ist, wobei die flügelartigen Profilkörper (7) zumindest im Bereich der Messstrecke angeordnet sind.

7. Messstab (1) nach einem der Ansprüche 1 bis 6, **dadurch gekennzeichnet, dass** wenigstens ein Störkörper (16) vorhanden ist, wobei der Störkörper (16) in Strömungsrichtung vor der Messstrecke angeordnet ist.

8. Messstab (1) nach einem der Ansprüche 1 bis7, **dadurch gekennzeichnet, dass** wenigstens ein Drucksensor (11), vorzugsweise ein Piezowandler, vorhanden ist, welcher innerhalb oder in Strömungsrichtung hinter der Messstrecke angeordnet ist.

9. Messstab (1) nach einem der Ansprüche 1 bis 8, **dadurch gekennzeichnet, dass** wenigstens ein Schutzring (8) zwischen dem rohrseitigen Ende und der Messstrecke angeordnet ist, wobei der Schutzring (8) den Umfang des Messstabs (1) ringförmig umgibt und wobei der Schutzring (8) einen Übertritt von Flüssigkeit von dem rohrseitigen Ende zur Messstrecke verhindert.

10. Messstab (1) nach einem der Ansprüche 1 bis 9, **dadurch gekennzeichnet, dass** im Bereich der Ausnehmung wenigstens ein Keramikfenster (9) zur Einkopplung und/oder zur Auskopplung zumindest des ersten Messsignals angeordnet ist.

11. Messstab (1) nach einem der Ansprüche 1 bis 10, **dadurch gekennzeichnet, dass** der Messstab (1) im Bereich der Ausnehmung (5) wenigstens einen Reflektor (10) aufweist, welcher derart angeordnet ist, dass zumindest das erste Messsignal (3) nach dem Durchlauf durch die Messstrecke in Richtung auf die Empfangseinheit (4) reflektiert wird.

12. Messstab (1) nach einem der Ansprüche 1 bis 11, **dadurch gekennzeichnet, dass** wenigstens eine zweite Sendeeinheit (12) für die Aussendung eines zweiten akustischen oder elektromagnetischen Messsignals (13) und wenigstens eine zweite Empfangseinheit (14) für den Empfang des zweiten Messsignals (13) vorhanden ist, wobei die zweite Sendeeinheit (12) derart angeordnet ist, dass das zweite Messsignal (13) die Messstrecke durchläuft, wobei die zweite Empfangseinheit (14) derart angeordnet ist, dass sie zumindest im strömungsfreien Betrieb das zweite Messsignal (13) nach dem Durchlauf der Messstrecke empfängt und wobei die zweite Sendeeinheit (12) und die zweite Empfangseinheit (14) in Strömungsrichtung hinter der ersten Sendeeinheit (12) und in Strömungsrichtung hinter der ersten Empfangseinheit (14) angeordnet sind.

13. Messstab (1) nach einem der Ansprüche 1 bis 12, **dadurch gekennzeichnet, dass** eine Auswerteeinheit (6) vorhanden ist, die zumindest mit der ersten Empfangseinheit (4) verbunden ist und wobei die Auswerteeinheit (6) die Amplitude und/oder die Phase und/oder die Intensität und/oder Laufzeit des Messsignals und/oder die Kreuzkorrelation des ersten und des zweiten Messsignals (3, 13) bestimmt.

14. Messanordnung (18) umfassend ein Rohr und einen Messstab (1) nach einem der Ansprüche 1 bis 13, wobei der Messstab (1) zumindest teilweise innerhalb des Rohrs angeordnet ist, wobei der Messstab von außen durch die Wandung des Rohres in den Strömungsquerschnitt eingebracht ist.

15. Messanordnung (18) nach Anspruch 14, **dadurch gekennzeichnet, dass** das Rohr einen Radius aufweist und dass der Messstab (1) höchstens um die Hälfte des Radius, vorzugsweise höchstens um ein Drittel des Radius in das Rohr hineinragt.

16. Messanordnung (18) nach einem der Ansprüche 14 oder 15, **dadurch gekennzeichnet, dass** ein weiterer Störkörper (17) vorhanden ist, wobei der Störkörper (17) in Strömungsrichtung vor dem Messstab (1) angeordnet ist.

## Claims

1. Measuring rod (1) with a longitudinal axis (A) for insertion in the flow cross section of a tube and for the verification of a flowing medium in this tube, comprising at least one first sender unit (2) for the transmission of a first acoustic or electromagnetic measuring signal (3) and at least one first receiver unit (4) for receiving the first measuring signal, wherein the first sender unit (2) and the first receiver unit (4) define a measuring section, wherein the first sender unit (2) is arranged in such a manner that the first measuring signal (3) crosses the measuring section and wherein the first receiver unit (4) is arranged in such a manner that it, at least during operation without flow, receives the first measuring signal (3) after crossing the measuring section,
wherein the measuring rod (1) has a recess (5) extending along its longitudinal axis (A),
**characterized in**
**that** at least one longitudinal surface (19) and two end faces (20, 21) are formed by the recess (5) in the measuring rod (1) and the measuring section is formed in the recess (5) between the two end faces (20, 21),
**that** a second longitudinal surface (22) is additionally formed by the recess (5) in the measuring rod (1), wherein the first longitudinal surface (19) and the second longitudinal surface (22) meet at a longitudinal edge (23) and comprise an angle and wherein, during operation of the measuring rod, the second longitudinal surface is arranged in front of the measuring section in terms of flow direction.

2. Measuring rod (1) according to claim 1, **characterized in that** the first sender unit (2) and the first receiver unit (4) are arranged at a distance to one another in respect to the longitudinal axis (A).

3. Measuring rod (1) according to claim 1 or 2, **characterized in that** an electric contact of the first sender unit (2) and the first receiver unit (4) runs within the measuring rod (1).

4. Measuring rod (1) according to any one of claims 1 to 3, **characterized in that** a third longitudinal surface is additionally formed by the recess (5) in the measuring rod (1), in particular wherein the first longitudinal surface (19), the second longitudinal surface (22) and the third longitudinal surface form a groove in the measuring rod (1).

5. Measuring rod (1) according to any one of claims 1 to 4, **characterized in that** at least one flow rectifier is provided for generating a rectified flow of the medium to be verified in the area of the measuring section.

6. Measuring rod (1) according to claim 5, **characterized in that** the flow rectifier is designed in the form of at least two wing-shaped profile bodies (7), wherein the wing-shaped profile bodies (7) are arranged at least in the area of the measuring section.

7. Measuring rod (1) according to any one of claims 1 to 6, **characterized in that** at least one bluff body (16) is provided, wherein the bluff body (16) is arranged before the measuring section in respect to flow direction.

8. Measuring rod (1) according to any one of claims 1 to 7, **characterized in that** at least one pressure sensor (11), preferably a piezo transducer, is provided, which is arranged within the measuring section or after it in respect to flow direction.

9. Measuring rod (1) according to any one of claims 1 to 8, **characterized in that** at least one protective ring (8) is arranged between the tube-side end and the measuring section, wherein the protective ring (8) annularly encompasses the circumference of the measuring rod (1) and wherein the protective ring (8) prevents a transgression of liquid from the tube-side end of the measuring section.

10. Measuring rod (1) according to any one of claims 1 to 9, **characterized in that** at least one ceramic window (9) is arranged in the area of the recess for coupling at least the first measuring signal in and/or out.

11. Measuring rod (1) according to any one of claims 1 to 10, **characterized in that**, in the area of the recess (5), the measuring rod (1) has at least one reflector (10) that is arranged in such a manner that at least the first measuring signal (3) is reflected toward the receiver unit (4) after crossing through the measuring section.

12. Measuring rod (1) according to any one of claims 1 to 11, **characterized in that** at least one second sender unit (12) is provided for transmission of a second acoustic or electromagnetic measuring signal (13) and at least one second receiver unit (14) is provided for receiving the second measuring signal (13), wherein the second sender unit (12) is arranged in such a manner that the second measuring signal (13) crosses the measuring section, wherein the second receiver unit (14) is arranged in such a manner that it, at least during operation without flow, receives the second measuring signal (13) after crossing the measuring section and wherein the second sender unit (12) and the second receiver unit (14) are arranged behind the first sender unit (2) in respect to flow direction and behind the first receiver unit (4) in respect to flow direction.

13. Measuring rod (1) according to any one of claims 1 to 12, **characterized in that** that an evaluation unit (6) is provided, which is connected at least to the first receiver unit (4) and wherein the evaluation unit (6) determines the amplitude and/or the phase and/or the intensity and/or the transit time of the measuring signal and/or the cross correlation of the first and the second measuring signals (3, 13).

14. Measuring arrangement (18) comprising a tube and a measuring rod (1) according to any one of claims 1 to 13, wherein the measuring rod (1) is arranged at least partially within the tube, wherein the measuring rod is introduced into the flow cross section from the outside through the wall of the tube.

15. Measuring arrangement (18) according to claim 14, **characterized in that** the tube has a radius and that the measuring rod (1) extends into the tube at no more than half of the radius, preferably at no more than one third of the radius.

16. Measuring arrangement (18) according to claim 14 or 15, **characterized in that** a further bluff body (17) is provided, wherein the bluff body (17) is arranged before the measuring rod (1) in terms of flow direction.

## Revendications

1. Jauge de mesure (1) comprenant un axe longitudinal (A) destinée à être introduite dans la section transversale d'écoulement d'un tube et pour l'identification d'un fluide s'écoulant dans ce tube, comprenant au moins une première unité d'émission (2) pour l'émission d'un premier signal de mesure (3) acoustique ou électromagnétique et au moins une première unité de réception (4) pour la réception du premier signal de mesure, la première unité d'émission (2) et la première unité de réception (4) définissant un segment de mesure, la première unité d'émission (2) étant disposée de telle sorte que le premier signal de mesure (5) traverse le segment de mesure et la première unité de réception (4) étant disposée de telle sorte qu'elle reçoit le premier signal de mesure (3) après la traversée du segment de mesure au moins dans un mode de fonctionnement sans écoulement,
la jauge de mesure (1) possédant un creux (5) qui s'étend le long de son axe longitudinal (A), **caractérisée en ce**
**qu'**au moins une première surface longitudinale (19) et deux surfaces frontales (20, 21) sont formées par le creux (5) dans la jauge de mesure (1) et le segment de mesure est formé dans le creux entre les deux surfaces frontales (20, 21),
**qu'**une deuxième surface longitudinale (22) est en plus formée dans la jauge de mesure (1) par le creux (5), la première surface longitudinale (19) et la deuxième surface longitudinale (22) se rencontrant au niveau d'une arête longitudinale (23) et incluant un angle et la deuxième surface longitudinale, en fonctionnement de la jauge de mesure, étant disposée avant le segment de mesure dans le sens de l'écoulement.

2. Jauge de mesure (1) selon la revendication 1, **caractérisée en ce que** la première unité d'émission (2) et la première unité de réception (4) sont disposées espacées l'une de l'autre en référence à l'axe longitudinal (A).

3. Jauge de mesure (1) selon la revendication 1 ou 2, **caractérisée en ce qu'**une mise en contact électrique de la première unité d'émission (2) et de la première unité de réception (4) suit un tracé à l'intérieur de la jauge de mesure (1).

4. Jauge de mesure (1) selon l'une des revendications 1 à 3, **caractérisée en ce qu'**une troisième surface longitudinale est formée par le creux (5) dans la jauge de mesure (1), la première surface longitudinale (19), la deuxième surface longitudinale (22) et la troisième surface longitudinale formant notamment une rainure dans la jauge de mesure (1).

5. Jauge de mesure (1) selon l'une des revendications 1 à 4, **caractérisée en ce qu'**au moins un redresseur d'écoulement destiné à produire un écoulement redressé du fluide à identifier est présent dans la zone du segment de mesure.

6. Jauge de mesure (1) selon la revendication 5, **caractérisée en ce que** le redresseur d'écoulement est réalisé sous la forme d'au moins deux corps profilés (7) de type aile, les corps profilés (7) de type aile étant disposés au moins dans la zone du segment de mesure.

7. Jauge de mesure (1) selon l'une des revendications 1 à 6, **caractérisée en ce qu'**au moins un corps perturbateur (16) est présent, le corps perturbateur (16) étant disposé avant le segment de mesure dans le sens de l'écoulement.

8. Jauge de mesure (1) selon l'une des revendications 1 à 7, **caractérisée en ce qu'**au moins un capteur de pression (11), de préférence un convertisseur piézoélectrique est présent, lequel est disposé à l'intérieur du segment de mesure ou derrière celui-ci dans le sens de l'écoulement.

9. Jauge de mesure (1) selon l'une des revendications 1 à 8, **caractérisée en ce qu'**au moins une bague de protection (8) est disposée entre l'extrémité côté tube et le segment de mesure, la bague de protection (8) entourant en forme d'anneau le pourtour de la jauge de mesure (1) et la bague de protection (8) empêchant un débordement de liquide de l'extrémité côté tube vers le segment de mesure.

10. Jauge de mesure (1) selon l'une des revendications 1 à 9, **caractérisée en ce qu'**au moins une fenêtre en céramique (9) servant à l'injection et/ou à l'extraction d'au moins le premier signal de mesure est disposée dans la zone du creux.

11. Jauge de mesure (1) selon l'une des revendications 1 à 10, **caractérisée en ce que** la jauge de mesure (1) possède au moins un réflecteur (10) dans la zone du creux (5), lequel est disposé de telle sorte qu'au moins le premier signal de mesure (3) est réfléchi en direction de l'unité de réception (4) après être passé à travers le segment de mesure.

12. Jauge de mesure (1) selon l'une des revendications 1 à 11, **caractérisée en ce qu'**au moins une deuxième unité d'émission (12) pour l'émission d'un deuxième signal de mesure (13) acoustique ou électromagnétique et au moins une deuxième unité de réception (14) pour la réception du deuxième signal de mesure (13) sont présentes, la deuxième unité d'émission (12) étant disposée de telle sorte que le deuxième signal de mesure (13) traverse le segment de mesure, la deuxième unité de réception (14) étant disposée de telle sorte qu'elle reçoit le deuxième signal de mesure (13) après la traversée du segment de mesure au moins dans un mode de fonctionnement sans écoulement et la deuxième unité d'émission (12) et la deuxième unité de réception (14) étant disposées derrière la première unité d'émission (12) dans le sens de l'écoulement et derrière la première unité de réception (14) dans le sens de l'écoulement.

13. Jauge de mesure (1) selon l'une des revendications 1 à 12, **caractérisée en ce qu'**une unité d'interprétation (6) est présente, laquelle est au moins reliée à la première unité de réception (4) et l'unité d'interprétation (6) déterminant l'amplitude et/ou la phase et/ou l'intensité et/ou le temps de propagation du signal de mesure et/ou la corrélation croisée du premier et du deuxième signal de mesure (3, 13) .

14. Arrangement de mesure (18) comprenant un tube et une jauge de mesure (1) selon l'une des revendications 1 à 13, la jauge de mesure (1) étant disposée au moins partiellement à l'intérieur du tube, la jauge de mesure étant introduite dans la section transversale d'écoulement depuis l'extérieur à travers la paroi du tube.

15. Arrangement de mesure (18) selon la revendication 14, **caractérisé en ce que** le tube possède un rayon et **en ce que** la jauge de mesure (1) fait saillie dans le tube de préférence au maximum d'un tiers du rayon.

16. Arrangement de mesure (18) selon l'une des revendications 14 ou 15, **caractérisé en ce qu'**un corps perturbateur (17) supplémentaire est présent, le corps perturbateur (17) étant disposé avant la jauge de mesure (1) dans le sens de l'écoulement.
